Europäisches Patentamt
European Patent Office
Office européen des brevets

(19)

(11) Veröffentlichungsnummer: **0 457 046 A1**

(12) # EUROPÄISCHE PATENTANMELDUNG

(21) Anmeldenummer: **91106152.1**

(51) Int. Cl.[5]: **C07D 233/61**, C08G 59/50

(22) Anmeldetag: **17.04.91**

(30) Priorität: **18.05.90 DE 4015961**

(43) Veröffentlichungstag der Anmeldung: **21.11.91 Patentblatt 91/47**

(84) Benannte Vertragsstaaten: **AT BE CH DE ES FR GB IT LI NL SE**

(71) Anmelder: **SCHERING AKTIENGESELLSCHAFT**
**Berlin und Bergkamen**
**Ernst-Schering-Strasse 14 Postfach 15 40**
**W-4709 Bergkamen(DE)**

(72) Erfinder: **Burba, Christian, Dipl.-Chem. Dr.**
**Gerhart-Hauptmann-Strasse 9**
**W-4715 Herbern(DE)**
Erfinder: **Mrotzek, Werner, Dipl.-Ing. Dr.**
**Dresdener Strasse 24**
**W-4600 Dortmund(DE)**

(54) **Imidazolylderivate, ihre Verwendung als Härtungsmittel in Epoxidharzzusammensetzungen, sie enthaltende härtbare Epoxidharzzusammensetzungen und Epoxidharzformkörper.**

(57) Gegenstand der Erfindung sind neue Verbindungen der allgemeinen Formeln (I) und (II)

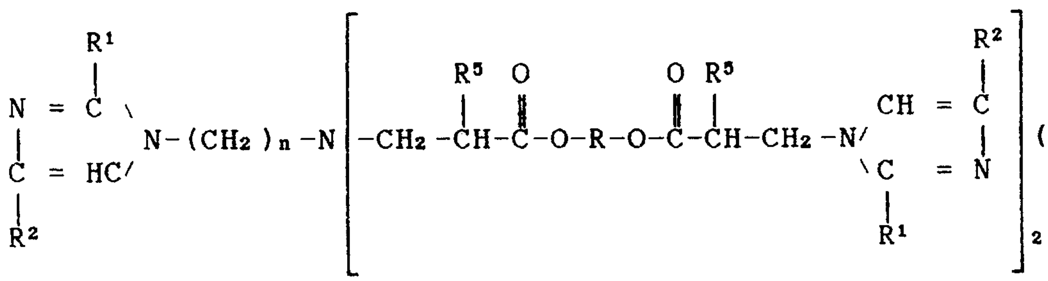

(I)

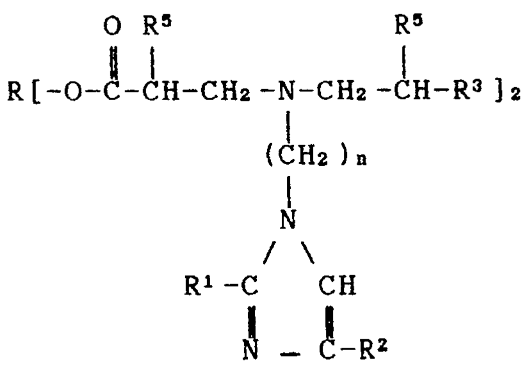

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN,

EP 0 457 046 A1

$-COOC_2H_4-OH$, $-CONH-NH_2$, $-COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann und ihre Verwendung als Härtungsmittel für Epoxidharze.

Gegenstand der Erfindung sind neue Imidazolylderivate, ihre Verwendung als Härtungsmittel in Epoxidharzzusammensetzungen, sie enthaltende härtbare Epoxidharzzusammensetzungen, bestehend aus einem Epoxidharz und aus den Verbindungen der allgemeinen Formel I und gegebenenfalls üblichen Härtungs- und Lösungsmitteln zur Herstellung von Formkörpern.

Für die Herstellung von Verbundwerkstoffen werden heute im Prinzip zwei Verfahren angewandt.

Bei dem Naß-in-Naß-Verfahren werden die Verstärkungsmaterialien mit der härtbaren Mischung imprägniert und in der Hitze in einer Stufe bis zum duromeren Endzustand ausgehärtet (Einstufenverfahren).

Beim Zweistufenverfahren werden aus den Verstärkungsmaterialien und der härtbaren Mischung zunächst sogenannte Prepregs hergestellt, die dann in einem zeitlich getrennten 2. Verfahrensschritt zu Fertigteilen weiterverarbeitet werden. Dabei ist verfahrenstechnisch noch einmal zwischen der Verwendung lösungsmittelhaltiger und lösungsmittelfreier Arbeitsweise zu unterscheiden.

Die Herstellung der Prepregs erfolgt normalerweise in einem kontinuierlichen Prozeß, in dem die Verstärkungsmaterialien entweder durch ein Imprägnierbad des einzusetzenden Harz-Härter-Gemisches geleitet werden oder das Imprägniermittel wird erst unmittelbar vor seinem Auftrag auf das Trägermaterial vermischt und mittels einer besonderen Vorrichtung aufgerakelt. Die Steuerung der auf eine bestimmte Trägermaterialbahn aufzubringenden Imprägniermittelmenge erfolgt dabei außer über die Viskosität des Imprägniermittels über nachgeschaltete Abquetschwalzen.

Bei lösungsmittelhaltigen Systemen wird nach dem Imprägnierprozeß durch Wärmezufuhr das in der Imprägnierlösung enthaltende Lösungsmittel verdampft und gleichzeitig das Harzsystem vom A- in den B-Zustand überführt. Aus den mit flüssigem bis stark klebrigem Imprägniermittel getränkten Verstärkungsmaterialien wird je nach Verfahrensbedingungen und verwendetem Harzsystem ein schwach klebriger bis fast trockener Prepreg. Bei diesem Verfahrensschritt ist es wichtig, daß einerseits das Lösungsmittel der Imprägniermischung vollständig entzogen wird, andererseits aber der für die Prepreg-Härtung im 2. Verfahrensschritt notwendige latente Härter noch nicht anspringt, um ein ungewolltes Ausreagieren der imprägnierten Verstärkungsmaterialien zu verhindern.

Bei lösungsmittelfreien Systemen erfolgt abhängig von der chemischen Zusammensetzung des Harzsystems nach der Imprägnierung entweder ebenfalls eine kurze Wärmebehandlung des Materials, oder die Verstärkungsstoffe werden unmittelbar nach der Imprägnierung unter Verzicht auf eine gesonderte Wärmebehandlung beidseitig mit Trennfolien kaschiert und einer systemadäquaten Zwischenlagerung zugeführt. Im Verlaufe dieser Zwischenlagerung tritt entweder ein allmählicher Übergang des Harzsystems in den B-Zustand ein oder das Imprägniermittel wird unter weitgehendem Verzicht auf chemische Veränderungen allein durch physikalische Effekte auf den Trägermaterialien fixiert.

Die so erhaltenen Prepregs lassen sich als Rollen zwischenlagern und transportieren, ehe sie dem jeweiligen Anwendungsfall entsprechend zugeschnitten und in Bauteildicke übereinander geschichtet werden. Durch gleichzeitige Druck- und Temperatureinwirkung wird der Prepregstapel zu einem hochfesten Formteil ausgehärtet, wobei die noch niedermolekularen, fließfähigen Harze in den hochmolekularen C-Zustand des Duromers übergehen.

Während beim Einstufenverfahren lange offene Zeiten und kurze Aushärtungszeiten bei niedrigen Härtungstemperaturen gefordert werden, kommt beim Zweistufenverfahren als weiteres Kriterium noch eine möglichst lange Lagerstabilität der Prepregs hinzu. Dabei werden Lagerungstemperaturen unterhalb Raumtemperatur von der Praxis immer weniger akzeptiert.

Von Bedeutung ist weiterhin, daß je nach Herstellungsverfahren der Prepregs die Materialviskosität der gebrauchsfertigen härtbaren Mischung über einen möglichst langen Zeitraum weitgehend unverändert bleibt. Speziell bei Verwendung eines großvolumigen Tränkbades ist dies für die Erzielung eines konstanten Harzauftrages und eines gleichbleibenden B-Zustandes erforderlich, da zum einen die Bedingungen der Produktion nicht laufend den sich ändernden Verhältnissen in der härtbaren Mischung angepaßt werden können und zum anderen dadurch auch die physikalischen Eigenschaften des ausgehärteten Endproduktes negativ beeinflußt werden.

Angestrebt wird in der Praxis eine härtbare Mischung, welche im Imprägnierbad auch über längere Zeit viskositätsmäßig konstant bleibt und dann als Prepreg bei Raumtemperatur ohne chemische Veränderungen lange gelagert werden kann.

Unabhängig von der Prepregherstellung soll ihre Aushärtung bei möglichst tiefen Temperaturen innerhalb kurzer Zeit erfolgen, das Temperaturmaximum der exothermen Reaktion auch bei größeren Schichtdicken auf einem niedrigen Niveau verbleiben und das physikalische Eigenschaftsniveau der Endprodukte den Erfordernissen der Praxis angepaßt sein.

Diese Forderungen hinsichtlich des Härtungsverhaltens und Eigenschaftsniveaus gelten in gleicher Weise auch für die im Naß-in-Naß-Verfahren zu verarbeitenden Epoxidharzsysteme.

Für gewisse Anwendungszwecke ist nur eine Anhärtung bis zur Formstabilität der Formkörper erforderlich bzw. sogar erwünscht, wobei nach einer eventuellen Zwischenlagerung die endgültige Aushärtung in einem nachgeschalteten Temperprozeß bei den erforderlichen Temperaturen erfolgt. Dabei ist es aber wichtig, daS bereits bei der Anhärtung die thermische Beständigkeit des Materials über die Härtungstemperatur steigt, da ansonsten die Entformung nur nach Absenkung der Formteiltemperatur möglich ist.

Das in härtbaren Mischungen auf Basis von Epoxidharzen seit langem als latenter Härter verwendete Dicyandiamid wird zur Erzielung der angestrebten Eigenschaften in der Regel mit Co-Härtungsmitteln und/oder Beschleunigern kombiniert. Aus der Literatur sind auf diesem Gebiet daher eine Vielzahl von Vorschlägen bekannt geworden.

Bei Verwendung von Dicyandiamidlösungen können zwar homogene Substrate hergestellt werden, jedoch bringt die Verwendung von Lösungsmitteln andere Probleme mit sich.

Dicyandiamid ist nur in wenigen Lösungsmitteln wie insbesondere Dimethylformamid oder Methylglykol in ausreichenden Mengen löslich. Diese Lösungsmittel sind jedoch toxikologisch bedenklich und bringen sowohl bei der Herstellung der Prepregs, das heißt bei Imprägnierung der Verstärkungsmaterialien und Überführung in den B-Zustand, als auch bei der Entsorgung Probleme mit sich.

Aufgrund der Schwerlöslichkeit des Dicyandiamids müssen größere Mengen an Lösungsmittel verwendet werden, welche die Tränkviskosität so beeinflussen, daß der Bindemittelgehalt auf den Verstärkungsmaterialien nicht beliebig gewählt werden kann.

Da diese Lösungsmittel bei der Härtung nicht vollständig entfernt werden können, besteht bei einer thermischen Belastung der Bauteile weiterhin die Gefahr, daß der Werkstoff vorzeitig versagt und/oder die Lösungsmittel vor Ort unkontrolliert an die Umgebungsluft abgegeben werden.

Bei Einsatz von festem kristallinen Dicyandiamid in flüssigen Epoxidharzen ohne Mitverwendung von Lösungsmitteln wird das Dicyandiamid entweder direkt in der erforderlichen Menge im Epoxidharz dispergiert oder es wird zuvor eine hochgefüllte Dicyandiamid/Epoxidharz-Paste hergestellt, welche zu einem späteren Zeitpunkt mit der Hauptmenge Epoxidharz auf die gewünschten Harz/Härter-Konzentrationen eingestellt wird.

In jedem Falle ist hier die Herstellung der Dispersionen nicht einfach, und außerdem neigen diese bei längeren Standzeiten insbesondere unter Imprägnierbedingungen zur Separation.

Bei Einsatz von festem kristallinen Dicyandiamid in bei Raumtemperatur festen Epoxidharzen ohne Mitverwendung von Lösungsmitteln wird in erster Stufe wiederum eine Paste aus Dicyandiamid und flüssigen Epoxidharzen hergestellt, welche dann bei erhöhter Temperatur in die Festharzschmelze eingearbeitet wird.

Neben den bereits oben angeführten Problemen werden bei dieser Vorgehensweise in das Festharz unverwünschte Anteile von flüssigen Epoxidharzen eingebracht.

Weiterhin werden bei Einsatz von festem kristallinen Dicyandiamid in den gehärteten Substraten Inhomogenitäten festgestellt, welche auf nicht gelösten und nicht umgesetzten Partikeln beruhen.

Aufgabe der vorliegenden Erfindung war es, unter Vermeidung der Nachteile des Standes der Technik, härtbare Mischungen auf Basis von Epoxidverbindungen und latenten und in den Epoxidharzen löslichen bzw. homogen verteilbaren Härtungsmitteln zu finden, welche bei relativ niedrigen Temperaturen innerhalb kurzer Zeit ohne hohe exotherme Temperaturspitzen zum formstabilen Zustand anhärten bzw. zum duromeren Endzustand aushärten, eine den Anforderungen der Praxis entsprechende thermische Beständigkeit besitzen und welche in Prepregs bei Raumtemperatur über eine ausreichende Lagerstabilität verfügen.

Diese Aufgabe wird gelöst durch Verwendung eines neuen Härtungsmittels gegebenenfalls unter Mitverwendung üblicher latenter Härtungsmittel.

Gegenstand der Erfindung sind daher Verbindungen der allgemeinen Formeln (I) und (II)

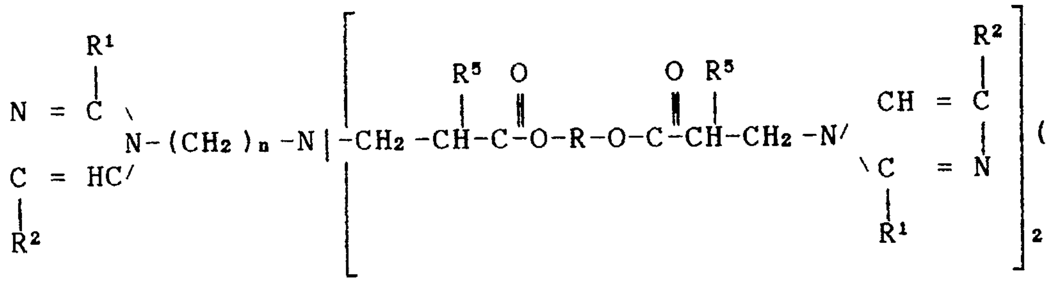 (I)

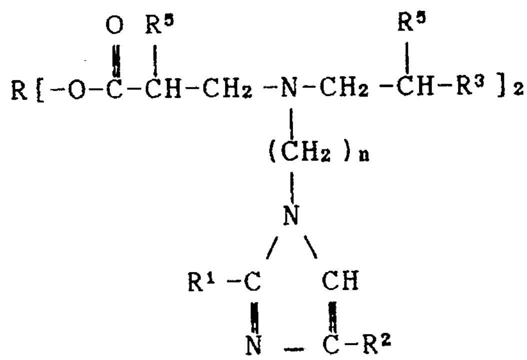 (II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20, insbesondere 4 - 8 C-Atomen ist, $R^1$ und $R^2$ unabhängig voneinander Wasserstoff, aliphatische oder aromatische Kohlenwasserstoffreste, insbesondere aber H-, $CH_3$-, $C_2H_5$-bedeuten, $R^3$ die Gruppen -COOH, -CN, -$COOC_2H_4$-OH, -CONH-$NH_2$, -$COOR^4$ sind mit $R^4$ = aliphatischer Kohlenwasserstoffrest mit 1 - 4 C-Atomen, insbesondere aber -$CH_2$-$CH_2$-OH ist und n = 2 oder 3 ist und $R^5$ Wasserstoff oder ein Methylrest sein kann.

Ein weiterer Gegenstand der Erfindung sind Härtungsmittel für Glycidylverbindungen, welche herstellbar sind durch Umsetzung von

A) Imidazolylverbindungen der allgemeinen Formel (III)

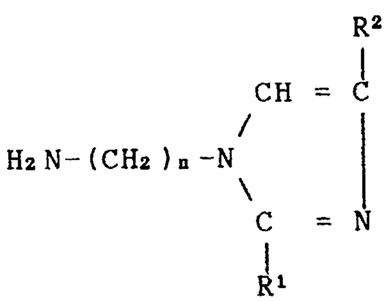 (III)

worin $R^1$ und $R^2$ unabhängig voneinander aliphatische oder aromatische Kohlenwasserstoffreste, insbesondere aber H-, $CH_3$, $C_2H_5$ - bedeuten und n = 2 und insbesondere = 3 ist mit

B) Diacrylaten der allgemeinen Formel (IV)

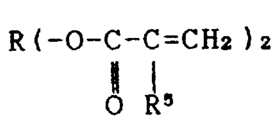 (IV)

worin R ein zweiwertiger, gegebenenfalls verzweigter aliphatischer, cyclischer oder alicyclischer Kohlenwasserstoffrest mit 2 - 20, insbesondere 4 - 8 C-Atomen ist und $R^5$ Wasserstoff oder ein Methylrest sein kann im Molverhältnis von A : B = 1 : 2 bis 2 : 1 und gegebenenfalls weiterer Umsetzung dieser Additionsverbindungen mit

(C)

1) Acrylsäure oder Acrylsäurederivaten der allgemeinen Formel (V)

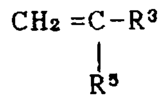

(V)

mit $R^3$ = -COOH, -CN, -CONH-$NH_2$, -$COOC_2H_4OH$, -$COOR^4$ mit $R^4$ = aliphatischer Kohlenwasserstoffrest mit 1 - 4 C-Atomen, wenn das Additionsprodukt aus A) und B) freie Aminwasserstoffatome aufweist oder gegebenenfalls mit

2) Imidazolen der allgemeinen Formel (VI)

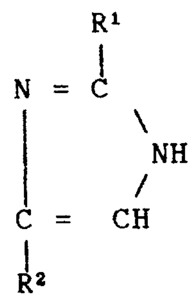

(VI)

in welcher $R^1$ und $R^2$ unabhängig voneinander die Bedeutung -H, $CH_3$-, $C_2H_5$, Phenyl haben können, wenn das Additionsprodukt aus A) und B) endständige Doppelbindungen (gemäß Formel IV) aufweist,

wobei die Verbindungen C) in solchen Mengen eingesetzt werden, daß alle reaktiven Aminwasserstoffatome oder Doppelbindungen der Additionsprodukte aus A) und B) umgesetzt werden.

Ein weiterer Gegenstand der Erfindung ist die Verwendung von Verbindungen der allgemeinen Formeln (I) und (II)

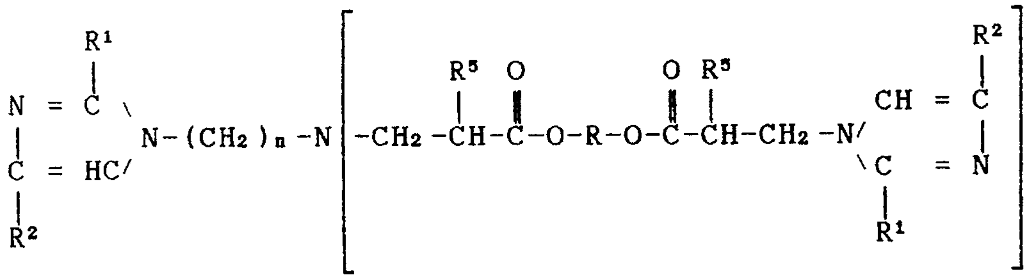

(I)

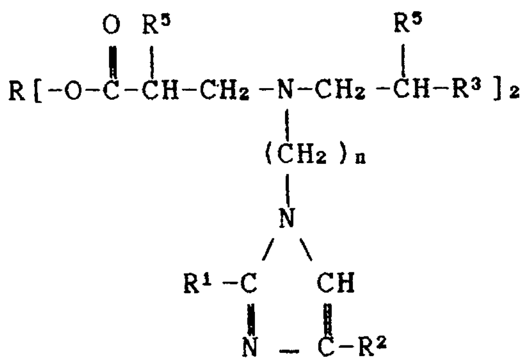

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20, insbesondere 4 - 8 C-Atomen ist, $R^1$ und $R^2$ unabhängig voneinander Wasserstoff, aliphatische oder aromatische Kohlenwasserstoffreste, insbesondere aber H-, $CH_3$-, $C_2H_5$-bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen, insbesondere aber $-CH_2-CH_2-OH$ ist und n = 2 oder 3 ist und $R^5$ Wasserstoff oder ein Methylrest sein kann, gegebenenfalls unter Mitverwendung üblicher stickstoffhaltiger heterocyclischer Aminverbindungen als Härtungsmittel für Epoxidharze.

Ein weiterer Gegenstand der Erfindung sind Epoxidharzformkörper, welche dadurch gekennzeichnet sind, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formeln (I) und (II)

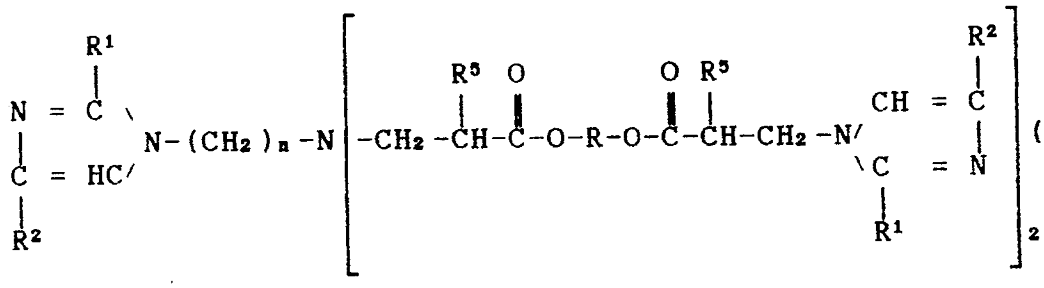

(I)

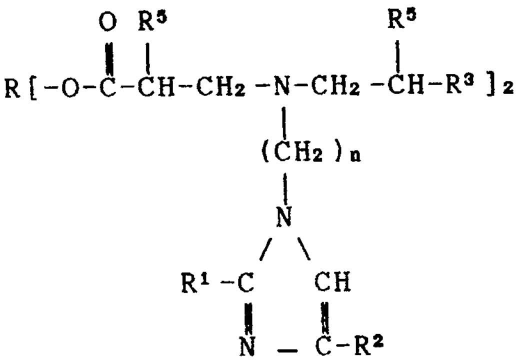

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20, insbesondere 4 - 8 C-Atomen ist, $R^1$ und $R^2$ unabhängig voneinander Wasserstoff, aliphatische oder aromatische Kohlenwasserstoffreste, insbesondere aber H-, $CH_3$-, $C_2H_5$)-bedeuten, $R^3$ die Gruppen -COOH, -CN, -$COOC_2H_4$,-OH, -CONH-$NH_2$, -$COOR^4$ sind mit $R^4$ = aliphatischer Kohlenwasserstoffrest mit 1 - 4 C-Atomen, insbesondere aber -$CH_2$-$CH_2$-OH ist und n = 2 oder 3 ist und $R^3$ H oder -$CH_3$ sein kann und gegebenenfalls

c) üblichen Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und in den festen und formstabilen Zustand überführt und in zweiter Stufe bei einer unterhalb der Erweichungstemperatur der gemäß Stufe 1 gebildeten Formkörper liegenden Temperatur ausgehärtet werden.

Weitere Gegenstände der Erfindung sind durch die Ansprüche gekennzeichnet.

Die erfindungsgemäßen Imidazolylverbindungen sind herstellbar durch Additionsreaktionen, wobei man in erster Stufe die Acrylatverbindungen mit primären Aminogruppen enthaltenden N-Aminoalkylimidazolyl-Verbindungen umsetzt, wobei das Verhältnis von Mol Acrylatverbindung zu Mol primären Aminogruppen 1 : 2 bis 2 : 1 betragen kann und in zweiter Stufe an die entstandenen sekundären Aminogruppen Acrylsäure bzw. Acrylsäurederivate und an die freien Doppelbindungen Imidazolylverbindungen der Formel VI addiert. Die Additionsreaktionen der ersten und zweiten Stufe werden nach den bekannten Verfahren durchgeführt.

Die erfindungsgemäß zur Herstellung der Additionsverbindungen mitverwendeten Acrylatverbindungen sind Ver- bzw. Umesterungsprodukte von mehrwertigen Alkoholen und Acrylsäuren. Diese Reaktionen erfolgen gemäß den zum bekannten Stand der Technik gehörenden Verfahren.

Als Alkohole können hier die geradkettigen oder verzweigten mehrwertigen aliphatischen Alkohole, insbesondere Diole, wie Butandiol, Hexandiol, Oktandiol, Neopentylglykol, Dekandiol oder cyclische oder alicyclische Diole wie Cyclohexandiol-(1,4) oder 1,4-Dihydroxymethyl-cyclohexan eingesetzt werden oder Ethergruppen enthaltende Diole wie Tetraethylenglykol, Tripropylenglykol.

Die erfindungsgemäß zur Herstellung der Additionsprodukte mitverwendeten Imidazolylverbindungen sind Verbindungen der allgemeinen Formel III

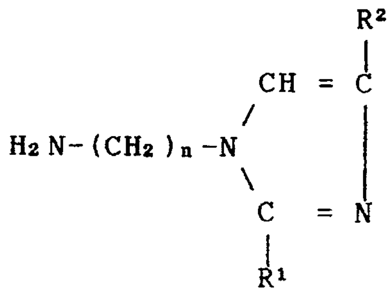

(III)

worin $R^1$ und $R^2$ unabhängig voneinander aliphatische oder aromatische Kohlenwasserstoff-Reste, insbesondere aber H-, $CH_3$, $C_2H_5$- bedeuten und n = 2 und insbesondere = 3 ist. Pro Mol Acrylatverbindung der oben angeführten Acrylatverbindungen (Formel IV) werden 1 - 2 Mol der Imidazolylverbindung der Formel III eingesetzt.

Die erfindungsgemäß mitverwendeten Acrylsäure- bzw. Acrylsäurederivate sind Verbindungen der allgmeinen Formel V

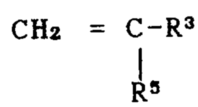

(V)

mit $R^3$ = -COOH, -CN, -CONH-$NH_2$, -$COOC_2H_4OH$, -$COOR_4$ mit $R^4$ = aliphatischer Kohlenwasserstoffrest mit 1 - 4 C-Atomen, insbesondere -$CH_3$ oder -$C_2H_5$.

Pro sekundärer Aminogruppe der in erster Stufe hergestellter Additionsverbindungen wird 1 Mol der Acrylsäureverbindungen eingesetzt.

Die erfindungsgemäß mitverwendeten, nur ein Aminwasserstoffatom aufweisenden Imidazolylverbindungen, welche an die in erster Stufe hergestellten Additionsverbindungen mit freien Doppelbindungen addiert werden, sind Verbindungen der allgemeinen Formel VI

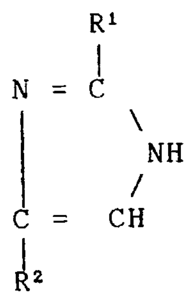

(VI)

in welcher $R^1$ und $R^2$ unabhängig voneinander die Bedeutung -H, $CH_3$, -$C_2H_5$, Phenyl haben können. Pro Doppelbindung wird 1 Mol der Verbindung gemäß Formel VI eingesetzt.

Die gemäß dieser Verfahrensschritte herstellbaren erfindungsgemäßen Additionsprodukte sind Verbindungen der allgemeinen Formeln I und II

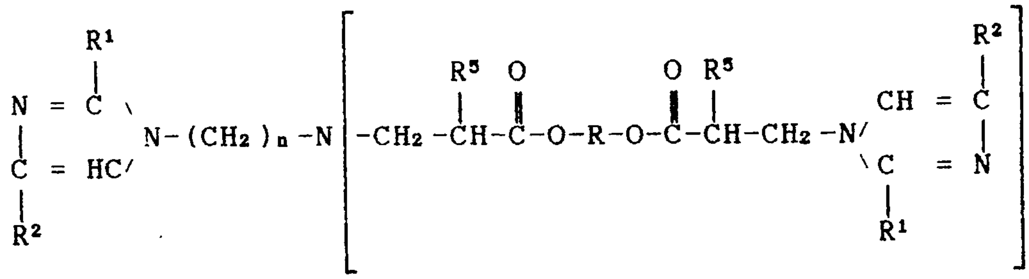

(I)

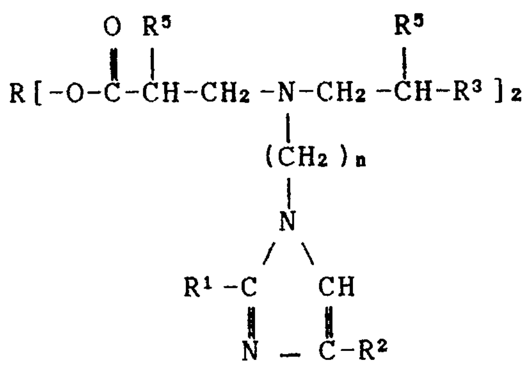

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20, insbesondere 4 - 8 C-Atomen ist, $R^1$ und $R^2$ unabhängig voneinander Wasserstoff, aliphatische oder aromatische Kohlenwasserstoffreste, insbesondere aber H-, $CH_3$-, $C_2H_5$- bedeuten, $R^3$ die Gruppen -COOH, -CN, -$COOC_2H_4$-OH, -CONH-$NH_2$, -$COOR^4$ sind mit $R^4$ = aliphatischer Kohlenwasserstoffrest mit 1 - 4 C-Atomen, insbesondere aber -$CH_2$-$CH_2$-OH ist und n = 2 oder 3 ist und $R^5$ Wasserstoff oder ein Methylrest sein kann.

Neben den bevorzugten Verbindungen der allgemeinen Formeln I und II, die dann entstehen, wenn das Verhältnis der Imidazolylverbindungen gemäß Formel III und den Glycidylverbindungen gemäß Formel IV geradzahlig 1 : 2 bzw. 2 : 1 ist, entstehen bei den zwischen diesen Werten liegenden Molverhältnissen Aufbauprodukte, welche die Struktureinheit

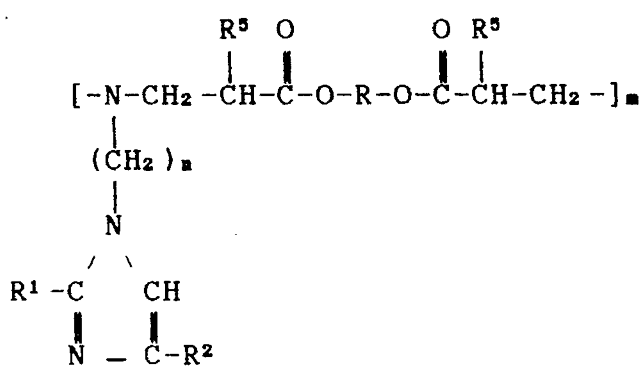

(VII)

mehrmals (m) in der Kette enthalten und entweder endständige Derivate der Verbindungen der Formeln V und VI aufweisen.

Sowohl m, welches vorzugsweise zwischen 1 - 5, insbesondere 1 - 3 liegt, als auch die Endgruppen können durch die Wahl der Molverhältnisse (Verbindungen Formel III : Verbindungen Formel IV) nach Wunsch bestimmt werden.

Dadurch ist es möglich, die katalytische Aktivität (tertiärer bzw. Gesamtstickstoffgehalt) zu steuern und die Viskosität von niedrigviskos über hochviskos bis fest einzustellen.

In erster Stufe werden die Additionsprodukte aus Imidazolylverbindungen der Formel III und den Acrylatverbindungen der Formel IV hergestellt, in Molverhältnissen, die sicherstellen, daß keine Doppelbindungen gleichzeitig neben tertiärem aliphatischen Stickstoff vorliegen, und in zweiter Stufe die Acrylsäurederivate gemäß Formel V addiert werden.

Bevorzugt wird jedoch so verfahren, daß alle Komponenten gleichzeitig zur Reaktion gebracht werden. Es wird dann ein statistisches Gemisch erhalten, welches in der Regel weniger einheitlich ist als das bei stufenweisem Vorgehen erhältliche. Dabei ist darauf zu achten, daß ein Überschuß an Acrylsäure (Derivaten) bei gleichzeitiger Anwesenheit von tertiärem Stickstoff vermieden wird. Ein wesentlicher Unterschied in der Brauchbarkeit für die erfindungsgemäß vorgesehenen Anwendungszwecke wurde nicht festgestellt.

Die erfindungsgemäßen Härtungsmittel können allein oder als Mischung eingesetzt werden, wobei pro 100 g Epoxidharz 2 - 35 g, vorzugsweise 4 - 25 g, insbesondere aber 5 - 20 g Härtungsmittel mitverwendet werden. Es ist ebenfalls möglich, die erfindungsgemäßen Imidazolyl-Verbindungen in Form ihrer Salze einzusetzen. Verwendbar sind hier die auf diesem Gebiet bekannten organischen und anorganischen Salzbildner. Bevorzugt werden erfindungsgemäß jedoch die ein- oder mehrwertigen organischen Carbonsäuren, wobei insbesondere die verzweigten Monocarbonsäuren mit bis zu 10 C-Atomen wie 2-Ethylhexansäure eingesetzt werden können.

Die erfindungsgemäß als Bindemittelbestandteil mitverwendeten Epoxidharze sind Glycidylester und -ether mit zwei oder mehr Epoxidgruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,2 - 0,6, insbesondere die bei Raumtemperatur flüssigen Verbindungen mit Epoxidwerten um 0,45 bis 0,55. Daneben haben sich auch die Glycidylether auf Basis von Bisphenol F und die Novolake als vorteilhaft erwiesen.

Die handelsüblichen halogenierten, insbesondere bromierten Epoxidharze auf Basis der oben aufgeführten Phenole sind ebenfalls verwendbar.

Als erfindungsgemäß gegebenenfalls mitzuverwendende Aminverbindungen können vorzugsweise übliche stickstoffhaltige heterocyclische Aminverbindungen, das heißt N-Alkylimidazole wie N-Methyl-, N-Ethylimidazol und/oder Imidzolinverbindungen der allgemeinen Formel (VIII)

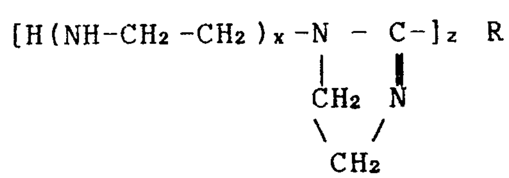

(VIII)

worin R ein gegebenenfalls verzweigter Alkyl- oder Alkylenrest mit < 10 C-Atomen, insbesondere $-CH_3$, $-CHOH-CH_3$, $-(CHR')-_y$, worin R' = H oder $-CH_3$ bedeuten und $_x$ = 1, 2, 3 und $_y$ = 4 - 8 und z gleich der Wertigkeit von R ist, mitverwendet werden, insbesondere solche, in denen $_x$ = 1, $_z$ = 1 und R = $-CH_3$ oder $-CH_2-CH_3$ ist. Andere auf diesem Gebiet übliche Härtungsmittel können, falls gewünscht, ebenfalls mitverwendet werden.

Zur Modifizierung der Eigenschaften des Endprodukts können neben anderen Epoxidharzen auch Modifizierungs- oder Hilfsstoffe mitverwendet werden wie Phenolharze, Melaminharze, Silikonharze, anorganische und organische Füllstoffe wie Quarzmehle, Titandioxid, Ruß, Silikon- oder Butadienkautschuk.

Zur Einstellung der gewünschten Viskosität können unterschiedlich viskose Harze oder Verdünnungsmittel mitverwendet werden, aber auch die üblichen Lösungsmittel wie Dimethylformamid, Aceton, Methylethylketon, Methylglykol, Propylenglykolmonomethylether oder deren Mischungen eingesetzt werden.

Zur Herstellung der Prepregs werden organische und anorganische Fasern, Vliese und Gewebe auf Basis von Aramid, Kohlenstoff oder Cellulose, Metallen wie Bor, Stahl usw., Keramik, insbesondere aber Glas, mitverwendet.

Die Herstellung der lösungsmittelhaltigen Prepregs geschieht nach der an sich bekannten Methode, bei der die Trägermaterialien in einem Imprägnierbad mit der reaktiven Harzmischung getränkt und nach dem Abquetschen der überschüssigen Harzmenge kontinuierlich unter Energiezufuhr (meist Wärme), bei gleichzeitigem Entzug des Lösungsmittels, vom A- in den B-Zustand überführt werden. Je nach gewünschter Prepreg-Konsistenz (klebrig bis fest) werden diese anschließend beidseitig mit einer Trennfolie versehen und für die Lagerung und den Transport aufgerollt. Die Weiterverarbeitung erfolgt durch Zuschneiden und Zusammenlegen der einzelnen Prepreg-Lagen zu einem Stapel, aus dem durch formgebende Maßnahmen

unter gleichzeitiger Wärmezufuhr ein hochvernetztes Werkstück entsteht.

Die erfindungsgemäßen Härtungsmittel können außerdem mit Erfolg in lösungsmittelfreien Prepregs auf Basis von Epoxidharzen und gegebenenfalls üblichen Härtungsmitteln verwendet werden. Hierbei werden die Trägermaterialien bei gegebebenenfalls erhöhter Temperatur nach an sich bekannten Verfahren mit den Bindemittelsystemen getränkt und systemadäquat gelagert, ehe sie wie die Lösungsmittel enthaltenden Systeme weiterverarbeitet werden.

Weitere Beispiele für lösungsmittelfreie Systeme sind Naßlaminate, Trägermaterialien für den Elektrosektor, insitu hergestellte faserverstärkte Formteile (RTM), heißhärtende Einkomponentenklebstoffe z. B. für die Verklebung von Karosserieteilen in der Automobilindustrie (Bördelnahtkleber) und Epoxidharzgießlinge, Epoxidharz-Beschichtungsmittel oder -wickelkörper.

Die in den Beispielen verwendeten Imidazolylverbindungen sowie die Acrylate sind handelsübliche Produkte der BASF Aktiengesellschaft, Ludwigshafen und wurden in dieser technischen Qualität eingesetzt.

## Beispiele

### I. Herstellung der erfindungsgemäßen Härter

#### Beispiel I.1

41,7 g (0,33 Mol) 1-(3-Aminopropyl)imidazol und 54,7 g (0,66 Mol) 2-Methylimidazol werden in 70,0 g Äthanol bei ca. 60 °C gelöst und mit 132,0 g (0,66 Mol) Butandioldiacrylat langsam versetzt. Man läßt 1 h bei 70 °C nachreagieren und zieht anschließend das Äthanol ab.

Man erhält ein Produkt mit folgenden Kennzahlen:

Aminzahl: 342/343
Viskosität/25 °C: 1,2 Pa.s

#### Beispiel I.2

250 g (2 Mol) 1-(3-Aminopropyl)imidazol werden vorgelegt und bei 60 °C bis 80 °C unter Kühlung mit 198 g (1 Mol) Butandioldiacrylat langsam versetzt.

Das Additionsprodukt hat folgende Kennzahlen:

Aminzahl: 494/497
Viskosität/25 °C: 1,6 Pa.s

448 g des oben beschriebenen Additionsproduktes werden bei 60 °c mit 232 g (2 Mol) 2-Hydroxyethylacrylat unter Kühlung langsam versetzt.

Man läßt 1 h bei 80 °C nachreagieren und erhält ein Produkt mit folgenden Kenndaten:

Aminzahl: 326/327
Viskosität/25 °C: 10,4 Pa.s

#### Beispiel I.3

375 g (3 Mol) 1-(3-Aminopropyl)imidazol werden vorgelegt und bei 60 °C bis 80 °C unter Kühlung mit 396 g (2 Mol) Butandioldiacrylat langsam versetzt.

Das Additionsprodukt hat folgende Kenndaten:

Aminzahl: 435/437
Viskosität/25 °C: 5,1 Pa.s

385,5 g des oben beschriebenen Additionsproduktes werden bei 60 °C mit 116 g (1 Mol) 2-Hydroxyethylacrylat unter Kühlung langsam versetzt.

Man läßt 1 h bei 80 °C nachreagieren und erhält ein Produkt mit folgenden Kenndaten:

Aminzahl: 335/337
Viskosität/25 °C : 6,7 Pa.s

#### Beispiel I.4

125 g (1 Mol) 1-(3-Aminopropyl)-imidazol und 164 g (2 Mol) 2-Methylimidazol werden in 150 g Äthanol bei ca. 60 °C gelöst und bei Temperaturen zwischen 60 und 80 °C mit 452,6 g (2 Mol) Hexandioldiacrylat langsam versetzt. Man läßt 1 h bei 70 °C nachreagieren und zieht anschließend das Äthanol ab. Dann erhält man ein Produkt mit folgenden Kennzahlen:

Aminzahl: 299/301
Viskosität/25 °C: 2,5 Pa.s

Beispiel I.5

300 g des Produktes gemäß I.4 werden mit 58,3 g 2-Ethylhexansäure bei 80 °C homogen verrührt. Man erhält ein Produkt mit folgenden Kennzahlen:
Aminzahl: 252
Viskosität/25 °C: 4,3 Pa.s

Beispiel I.6

125 g (1 Mol) 1-(3-Aminopropyl)-imidazol werden vorgelegt und bei Temperaturen zwischen 60 °C - 80 °C 198 g (1 Mol) Butandioldiacrylat langsam zugegeben. Nach Abklingen der exothermen Reaktion wird 3 h bei 90 °C nachgerührt. Man erhält ein Produkt mit folgenden Kennzahlen:
Aminzahl: 345/346
Viskosität/25 °C: 1290 mPa.s

II. Herstellung einer Prepreg-Reaktionsmischung

Beispiel 1

100 g eines Epoxidharzes (Epoxid-Äquivalentgewicht ca. 190) werden mit 14 g des erfindungsgemäßen Reaktionsprodukts I.1 vermischt und zur Prepreg-Herstellung eingesetzt. Diese Mischung besitzt bei Raumtemperatur (25 °C) eine Viskosität von 11,6 Pa.s und ist auch nach 10 h noch verarbeitungsfähig.

Die Herstellung der Prepregs im Labormaßstab erfolgt durch Aufstreichen des Reaktionsgemisches auf ein ca. 0,1 $m^2$ großes Glasfilamentgewebe in Atlasbindung, welches nach der Imprägnierung beidseitig mit Trennfolien kaschiert und bei Raumtemperatur gelagert wird.

Nach einer Zwischenlagerung von 24 h bei Raumtemperatur ist das Material soweit gereift, daß es als schwach klebriger Prepreg in mehreren Lagen im Heißpreßverfahren bei 0,1 bar und Temperaturen von 100 - 120 °C in 30 min bis 1 h zu hochfesten Formteilen weiterverarbeitet werden kann. Das derart ausgehärtete Endprodukt zeigt keinerlei Mängel bezüglich der Haftung der einzelnen Prepreglagen.

Die Ermittlung der in Tabelle 1 angegebenen Lagerstabilität erfolgt in Anlehnung an praxisgerechte Bedingungen. Das imprägnierte Gewebe wird zwischen zwei Polyethylenfolien bei 23 °C unter Normklima gelagert. In Abständen von 24 h wird eine Lage einer Probe unter praxisgerechten Bedingungen (1 h, 120 °C, 0,1 bar) verpreßt. Als Lagerstabilität wird der Tag bestimmt, an dem das Harz letztmalig unter Preßbedingungen fließfähig ist. Analog Beispiel 1 sind die weiteren in der Tabelle 1 angeführten Lagerstabilitäten ermittelt.

Tabelle 1 Lagerstabilitäten lösungsmittelfreier Prepregs

| Beispiel | Härtungsmittel | g Härtungs-mittel | 100 g Epoxid-harz | Lagerstabili-tät (Tage) |
|---|---|---|---|---|
| 1 | I.1 | 14 | Bisphenol A-Typ Ep-Wert 0,53 | > 6 |
| 2 | I.2 | 5 | " | > 25 |
| 3 | I.3 | 5 | " | > 6 |
| 4 | I.4 | 14 | " | > 6 |
| 5 | I.5 | 14 | " | > 6 |

III. Ermittlung der Härtungsmitteleinflüsse

Zur Ermittlung der Härtungsmitteleigenschaften in Abhängigkeit der jeweiligen Struktur werden zur Vermeidung verfälschender Einflüsse von Verstärkungs- und Zusatzmaterialien die Mischungen, bestehend allein aus Epoxidharz und Härtungsmittel, ausgehärtet und abgeprüft.

In den in der Tabelle 2 aufgelisteten Beispielen wird als Epoxidharz ein Glycidylether auf Basis von Bisphenol A mit einem Epoxidwert von 0,53 verwendet.

Zur Herstellung der Prüfkörper werden jeweils 100 g des Epoxidharzes mit der in der Tabelle 1 genannten Menge Härtungsmittel bei Raumtemperatur vermischt und in einem Stahlwerkzeug 2 h bei 120 °C zu 4 mm dicken ebenen Formteilen ausgehärtet. Aus diesen Formteilen werden dann durch Sägen bzw. Fräsen Probekörper entnommen, an denen unter Einhaltung der nachstehend genannten Prüfnormen die in Tabelle 2 aufgeführten Eigenschaftswerte ermittelt sind.

| | | Prüfkörperabmessungen: |
|---|---|---|
| Biegefestigkeit | DIN 53 452 | $80 \times 10 \times 4$ mm³ |
| Durchbiegung | DIN 53 452 | $80 \times 10 \times 4$ mm³ |
| Schlagzähigkeit | DIN 53 453 | $50 \times 6 \times 4$ mm³ |
| Zugfestigkeit | DIN 53 455 | Schulterstab Nr. 3 |
| Dehnung | DIN 53 455 | Schulterstab Nr. 3 |
| Elastizitätsmodul | DIN 53 457 | Schulterstab Nr. 3 |

| | | |
|---|---|---|
| Heat Distortion Temperature (HDT) | DIN 53 461 | Prüfkörperabmessungen $120 \times 10 \times 4$ mm³ |
| Glasübergangs-temperatur (TG) | DIN 53 445 | Prüfkörperabmessungen $80 \times 10 \times 1$ mm³ |

Tabelle 2 Thermische und mechanische Eigenschaften

| Beispiel | | 1 | 2 | 3 | 4 | 5 |
|---|---|---|---|---|---|---|
| Biegefestigkeit | N/mm² | 93 | 45 | 26 | 82 | 78 |
| Durchbiegung | mm | 13,7 | 4,3 | 4,8 | 8,3 | 7,8 |
| Schlagzähigkeit | kJ/m² | 12,5 | 3,0 | 3,1 | 8,5 | 5,5 |
| Zugfestigkeit | N/mm² | 50 | 31 | 34 | 41 | 43 |
| Dehnung | % | 2,2 | 1,2 | 1,2 | 1,7 | 1,8 |
| Elastizitätsmodul | N/mm² | 2800 | 2850 | 2970 | 2850 | 2740 |
| HDT | °C | 127 | 127 | 127 | 127 | 122 |
| TG | °C | 153 | 163 | 151 | 153 | 149 |

**Patentansprüche**

**1.** Verbindungen der allgemeinen Formeln (I) und (II)

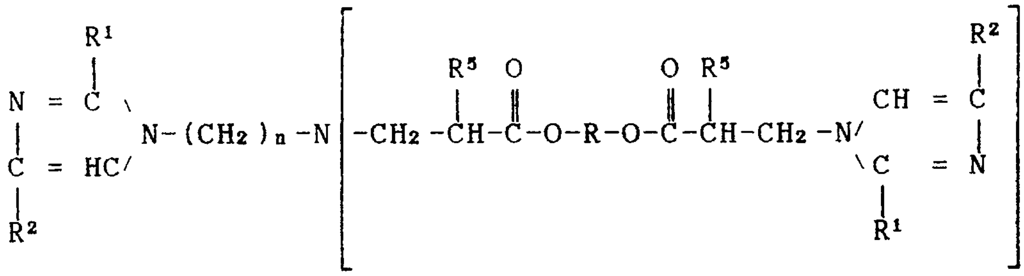 (I)

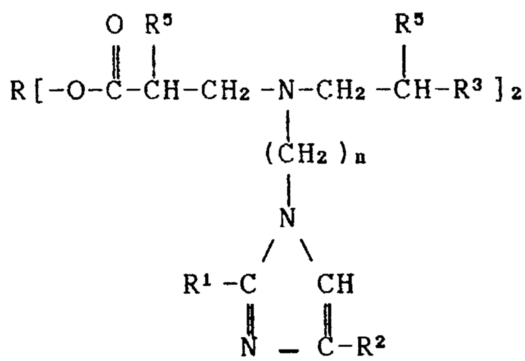 (II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen $-COOH$, $-CN$, $-COOC_2H_4-OH$, $-CONH-NH_2$, $-COOR^4$ sind mit $R^4$ = aliphatischer Kohlenwasserstoffrest mit 1 - 4 C-Atomen und n = 2 oder 3 ist und $R^5$ Wasserstoff oder ein Methylrest sein kann.

**2.** Verwendung von Verbindungen der allgemeinen Formeln (I) und (II)

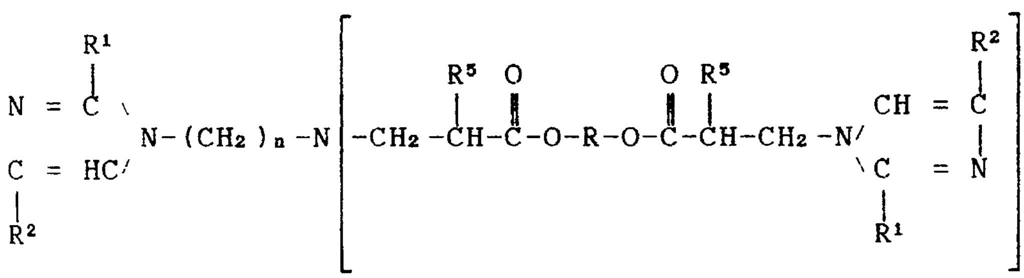 (I)

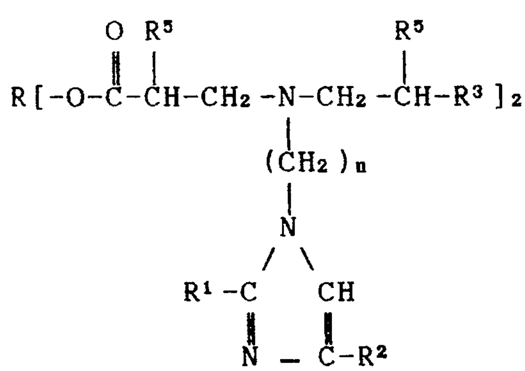 (II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen und n = 2 oder 3 ist und $R^5$ Wasserstoff oder ein Methylrest sein kann, als Härtungsmittel für Epoxidharze.

3. Härtungsmittel für Glycidylverbindungen, welche herstellbar sind durch Umsetzung von

A) Imidazolylverbindungen der allgemeinen Formel (III)

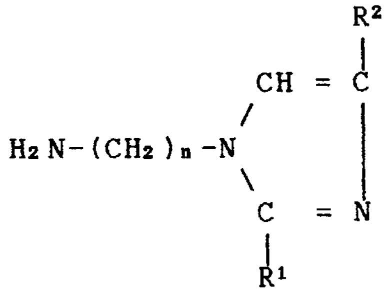

(III)

worin $R^1$ und $R^2$ unabhängig voneinander Wasserstoff, aliphatische oder aromatische Kohlenwasserstoffreste bedeuten und n = 2 oder 3 ist,

B) Diacrylaten der allgemeinen Formel (IV)

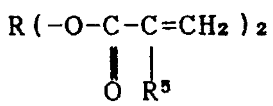

(IV)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist und $R^3$ Wasserstoff oder ein Methylrest sein kann im Molverhältnis von A : B = 1 : 2 bis 2 : 1 und gegebenenfalls weiterer Umsetzung dieser Additionsverbindungen mit

C)

1) Acrylsäure oder Acrylsäurederivaten der allgemeinen Formel (V)

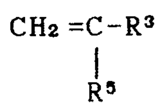

(V)

mit $R^3$ = -COOH, -CN, $-CONH-NH_2$, $-COOC_2H_4OH$, $-COOR^4$ mit $R^4$ = aliphatischer Kohlenwasserstoffrest mit 1 - 4 C-Atomen und $R^5$ Wasserstoff oder ein Methylrest sein kann, wenn das Additionsprodukt aus A) und B) freie Aminwasserstoffatome aufweist oder gegebenenfalls mit

2) Imidazolen der allgemeinen Formel (VI)

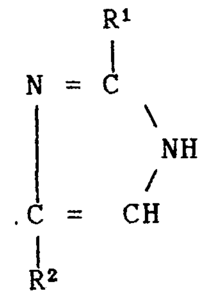

(VI)

in welcher $R^1$ und $R^2$ unabhängig voneinander die Bedeutung -H, $CH_3$ -, $C_2H_5$, Phenyl haben können, wenn das Additionsprodukt aus A) und B) freie Doppelbindungen aufweist,

wobei die Verbindungen C) in solchen Mengen eingesetzt werden, daß alle reaktiven Aminwasserstoffatome oder Epoxidgruppen der Additionsverbindungen aus A) und B) umgesetzt werden.

**4.** Härtbare Epoxidharz-Zusammensetzungen enthaltend

a) ein Epoxidharz mit durchschnittlich mehr als einer Epoxidgruppe pro Molekül und

b) Verbindungen der allgemeinen Formeln (I) und (II)

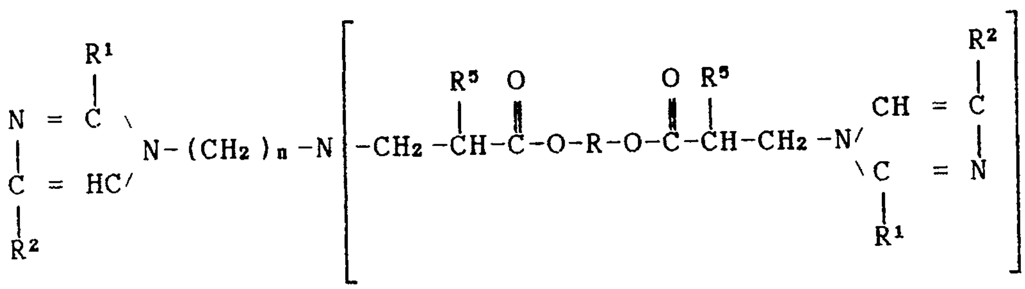

(I)

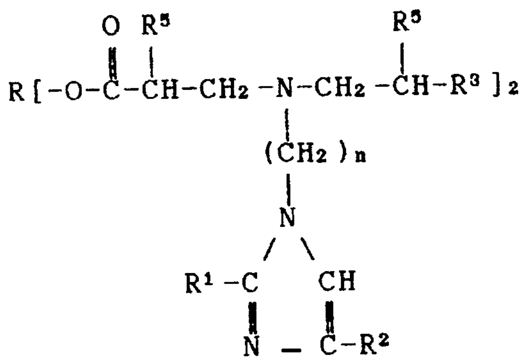

(II)

worin R ein zweiwertiger, gegebenenfalls verzweigter, gegebenenfalls Ethergruppen enthaltender aliphatischer` cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann

c) übliche Lösungsmittel, Füllstoffe, Verstärkungs- oder Einlagematerialien, Pigmente, Hilfsstoffe und gegebenenfalls

d) übliche stickstoffhaltige heterocyclische Aminverbindungen.

**3.** Härtbare Epoxidharz-Zusammensetzungen, worin die Verstärkungs- oder Einlagematerialien bei Raumtemperatur mit dem Bindemittel, bestehend aus

a) einem Epoxidharz mit durchschnittlich mehr als einer Epoxidgruppe pro Molekül und b) Verbindungen der allgemeinen Formeln (I) und (II)

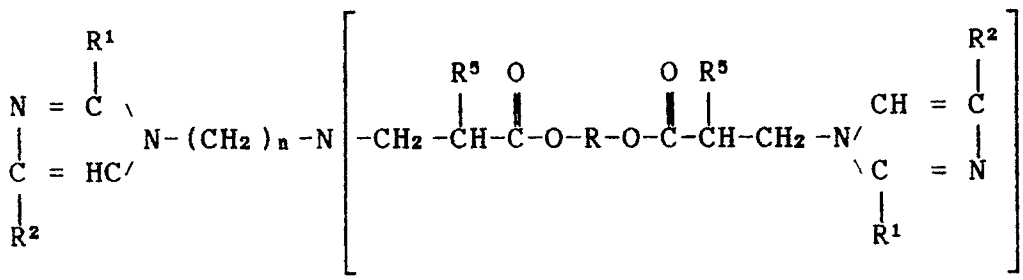

(I)

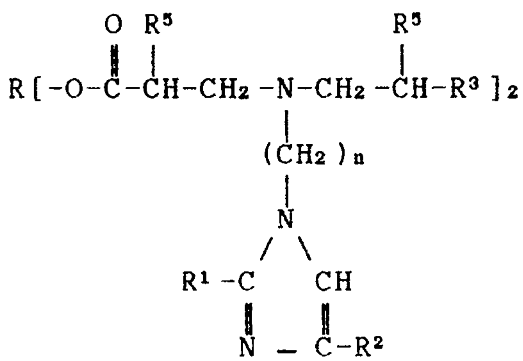

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste, $CH_3$-, $C_2H_5$- bedeuten, $R^3$ die Gruppen -COOH, -CN, $-COOC_2H_4-OH$, $-CONH-NH_2$, - $COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und gegebenenfalls bei erhöhter Temperatur in den halbfesten aber noch schmelzbaren Zustand (B-Zustand) überführt werden.

**6.** Härtbare Epoxidharz-Zusammensetzungen, worin Verstärkungs- oder Einlagematerialien bei Raumtemperatur mit dem Bindemittel, bestehend aus

a) einem Epoxidharz mit durchschnittlich mehr als einer Epoxidgruppe pro Molekül und

b) Imidazolylverbindungen, welche herstellbar sind durch Umsetzung von

A) Imidazolylverbindungen der allgemeinen Formel (III)

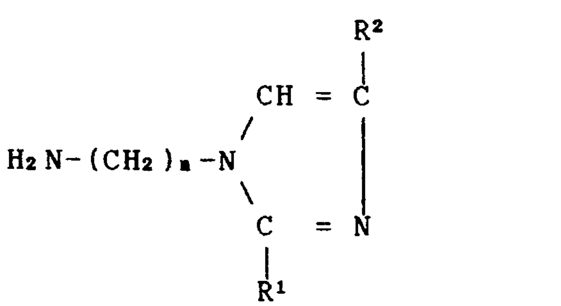

(III)

worin $R^1$ und $R^2$ unabhängig voneinander Wasserstoff, aliphatische oder aromatische Kohlenwasserstoffreste bedeuten und n = 2 oder 3 ist und

B) Diacrylaten der allgemeinen Formel (IV)

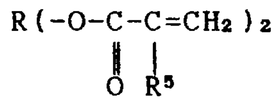

(IV)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist und $R^5$ Wasserstoff oder ein Methylrest sein kann im Molverhältnis von A : B = 1 : 2 bis 2 : 1 und gegebenenfalls weiterer Umsetzung dieser Additionsverbindungen mit

C)

1) Acrylsäure oder Acrylsäurederivaten der allgemeinen Formel (V)

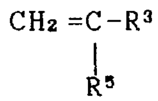

(V)

mit $R^3$ = $-COOH$, $-CN$, $-CONH-NH_2$, $-COOC_2H_4OH$,$-COOR^4$ mit $R^4$ = aliphatischer Kohlenwasserstoffrest mit 1 - 4 C-Atomen und $R^5$ Wasserstoff oder ein Methylrest sein kann, wenn das Additionsprodukt aus A) und B) freie Aminwasserstoffatome aufweist oder gegebenenfalls mit

2) Imidazolen der allgemeinen Formel (VI)

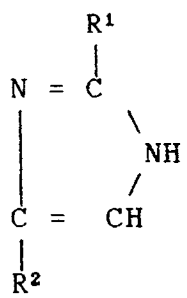

(VI)

in welcher $R^1$ und $R^2$ unabhängig voneinander die Bedeutung -H, $CH_3$-, $C_2H_5$, Phenyl haben können, wenn das Additionsprodukt aus A) und B) freie Doppelbindungen aufweist, wobei die Verbindungen C) in solchen Mengen eingesetzt werden, daß alle reaktiven

Aminwasserstoffatome oder Doppelbindungen der Additionsverbindungen aus A) und B) umgesetzt werden und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen.

getränkt und gegebenenfalls bei erhöhter Temperatur in den halbfesten aber noch schmelzbaren Zustand (B-Zustand) überführt werden.

**7.** Epoxidharzformkörper, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formeln (I) und (II)

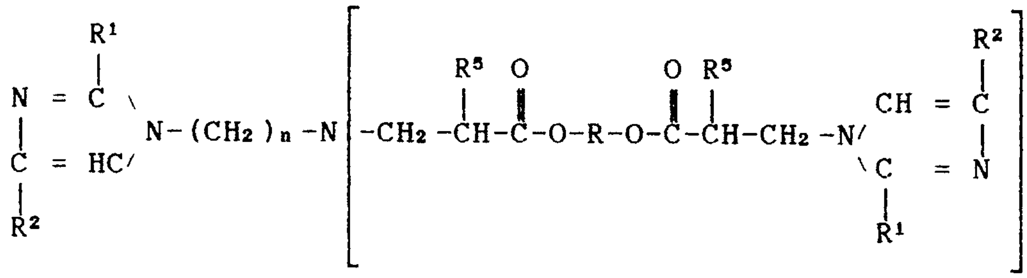

(I)

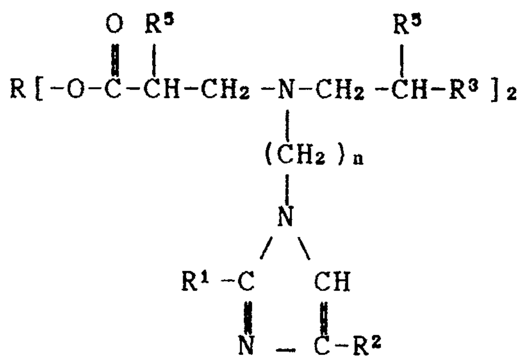

(II)

worin R ein zweiwertiger, gegebenenfalls verzweigter, gegebenenfalls Ethergruppen enthaltendcer aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann und gegebenenfalls

c) üblichen Lösungsmitteln, Füllstoffen, Verstärkungs- und Einlagematerialien, Pigmenten, Hilfsstoffen und gegebenenfalls

d) üblichen stickstoffhaltigen heterocyclischen Aminverbindungen

getränkt und gegebenenfalls in den halbfesten aber noch schmelzbaren Zustand (B-Zustand) überführt werden und in zweiter Stufe die feuchten Laminate oder Prepregs unter Formgebung oder zwischen zu verklebende Substrate gebracht, unter Anwendung von Druck bei erhöhter Temperatur ausgehärtet werden.

**8.** Epoxidharzformkörper, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formel (I) und (II)

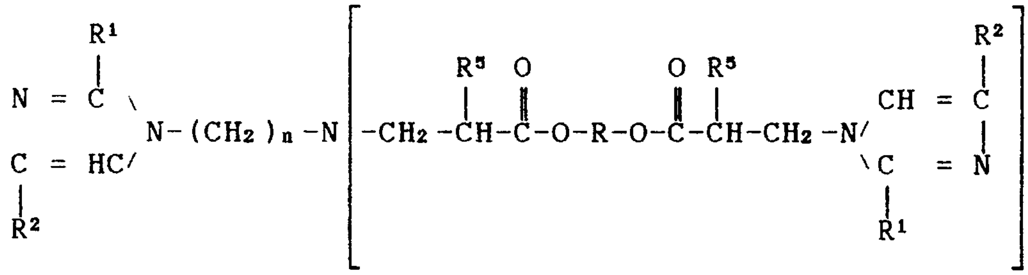

(I)

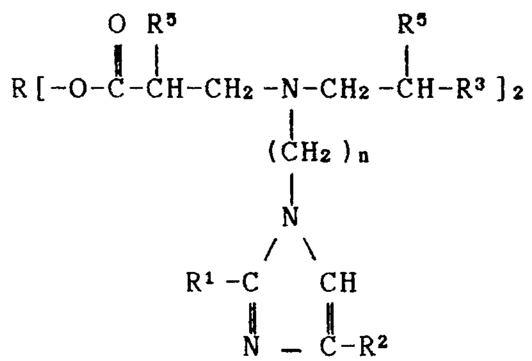

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen ist und n = 2 oder 3 ist und $R^3$ Wasserstoff oder ein Methylrest sein kann und gegebenenfalls

c) übliche Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und in den festen und formstabilen Zustand überführt und in zweiter Stufe bei einer unterhalb der Erweichungstemperatur der gemäß Stufe 1 gebildeten Formkörper liegenden Temperatur ausgehärtet werden.

**9.** Epoxidharzformkörper, hergestellt gemäß Anspruch 8, dadurch gekennzeichnet, daß als Härtungsmittel die Verbindungen gemäß Anspruch 3 eingesetzt werden.

**10.** Verfahren zur Herstellung von faserverstärkten Trägermaterialien für den Elektrosektor durch Überführung von mit Bindemittel auf Basis von Epoxidharz und aminischen Härtungsmitteln imprägnierten Verstärkungsmaterialien in erster Stufe durch Anwendung von Temperatur und gegebenenfalls Druck in den B-Zustand und endgültiger Aushärtung bei erhöhter Temperatur, dadurch gekennzeichnet, daß als Härtungsmittel Verbindungen der allgemeinen Formeln (I) und (II)

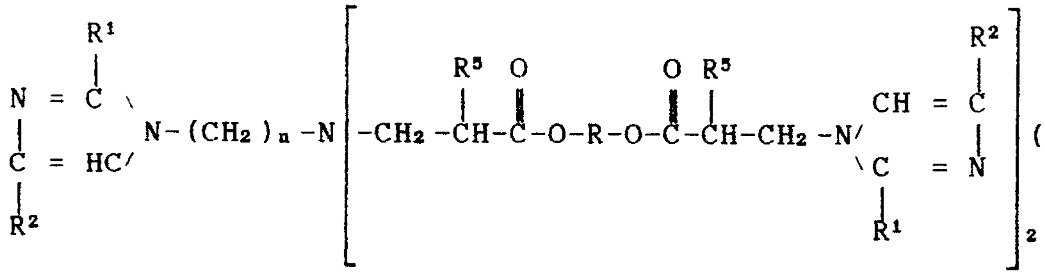

(I)

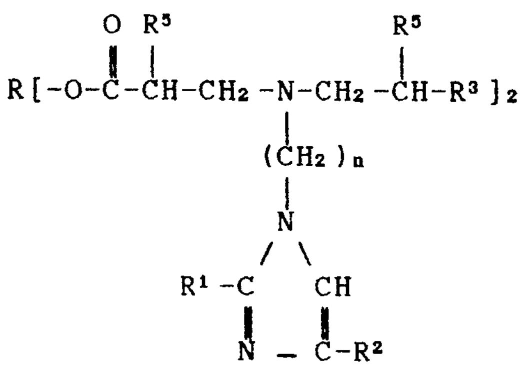

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste $C_2H_5$- bedeuten, $R^3$ die Gruppen -COOH, -CN, -$COOC_2H_4$-OH, -CONH-$NH_2$, -$COOR^4$ sind mit $R^4$ aliphatischer Kohlenwasserstoffrest mit 1 - 4 C-Atomen ist und n = 2 oder 3 ist und $R^5$ Wasserstoff oder ein Methylrest sein kann, verwendet werden.

**Patentansprüche für folgenden Vertragsstaat: ES**

1. Verfahren zur Hestellung von Härtungsmitteln für Glycidylverbindungen der allgemeinen Formeln (I) und (II)

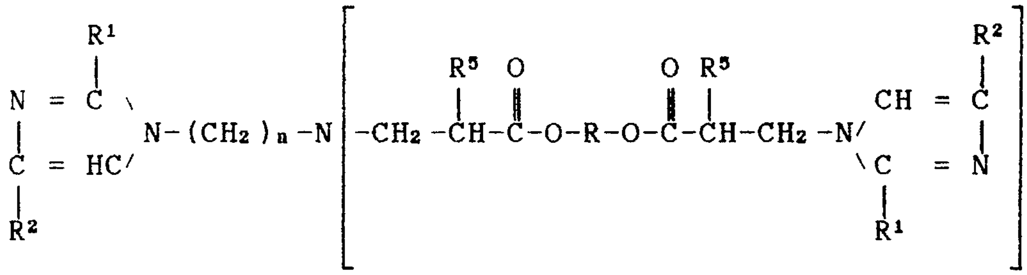

(I)

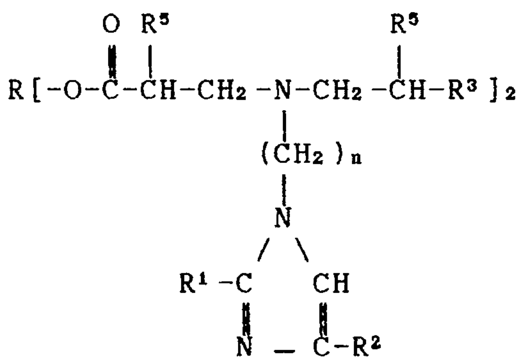

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen und n = 2 oder 3 ist und $R^5$ Wasserstoff oder ein Methylrest sein kann, durch Umsetzung von

A) Imidazolylverbindungen der allgemeinen Formel (III)

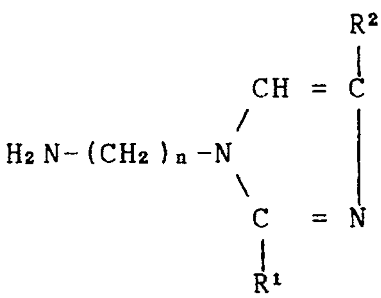

(III)

worin $R^1$ und $R^2$ unabhängig voneinander Wasserstoff, aliphatische oder aromatische Kohlenwasserstoff-Reste bedeuten und n = 2 oder 3 ist,

B) Diacrylaten der allgemeinen Formel (IV)

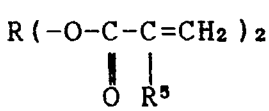

(IV)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist und $R^5$

Wasserstoff oder ein Methylrest sein kann im Molverhältnis von A : B = 1 : 2 bis 2 : 1 und gegebenenfalls weiterer Umsetzung dieser Additionsverbindungen mit

C)

1) Acrylsäure oder Acrylsäurederivaten der allgemeinen Formel (V)

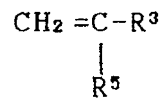

(V)

mit $R^3$ = -COOH, -CN, -CONH-$NH_2$, -$COOC_2H_4OH$,-$COOR^4$ mit $R^4$ = aliphatischer Kohlenwasserstoffrest mit 1 - 4 C-Atomen und $R^5$ Wasserstoff oder ein Methylrest sein kann, wenn das Additionsprodukt aus A) und B) freie Aminwasserstoffatome aufweist oder gegebenenfalls mit

2) Imidazolen der allgemeinen Formel (VI)

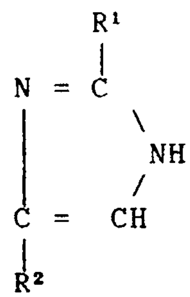

(VI)

in welcher $R^1$ und $R^2$ unabhängig voneinander die Bedeutung -H, $CH_3$-, $C_2H_5$, Phenyl haben können, wenn das Additionsprodukt aus A) und B) freie Doppelbindungen aufweist,

wobei die Verbindungen C) in solchen Mengen eingesetzt werden, daß alle reaktiven Aminwasserstoffatome oder Epoxidgruppen der Additionsverbindungen aus A) und B) umgesetzt werden.

**2.** Verwendung von Verbindungen der allgemeinen Formeln (I) und (II)

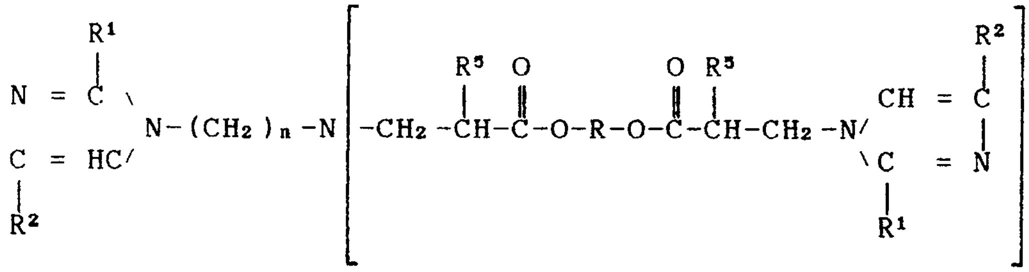

(I)

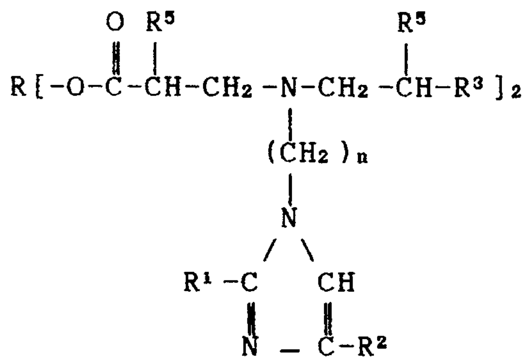

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen und n = 2 oder 3 ist und $R^5$ Wasserstoff oder ein Methylrest sein kann, als Härtungsmittel für Epoxidharze.

**3.** Verfahren zur Herstellung härtbarer Epoxidharzzusammensetzungen, worin die Verstärkungs- oder Einlagematerialien bei Raumtemperatur mit dem Bindemittel, bestehend aus

a) einem Epoxidharz mit durchschnittlich mehr als einer Epoxidgruppe pro Molekül und

b) Verbindungen der allgemeinen Formeln (I) und (II)

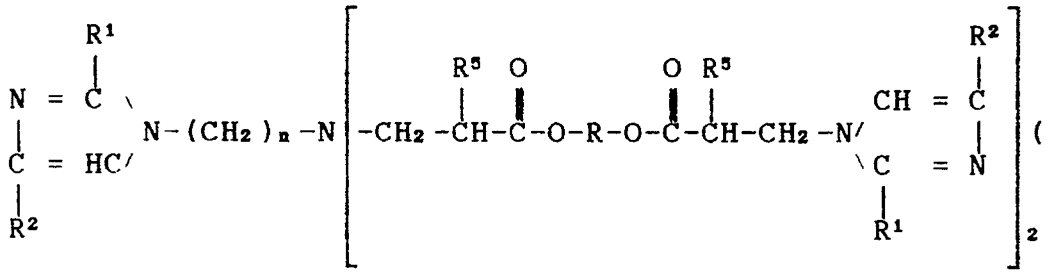

(I)

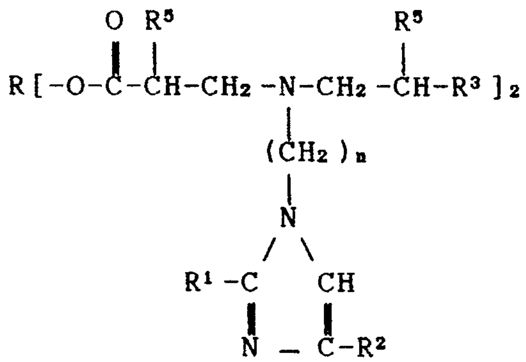

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste, $CH_3$-, $C_2H_5$- bedeuten, $R^3$ die Gruppen -COOH, -CN, $-COOC_2H_4-OH$, $-CONH-NH_2$, - $COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann, und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und gegebenenfalls bei erhöhter Temperatur in den halbfesten aber noch schmelzbaren Zustand (B-Zustand) überführt werden.

**4.** Verfahren zur Herstellung härtbarer Epoxidharz-Zusammensetzungen, worin Verstärkungs- oder Einlagematerialien bei Raumtemperatur mit dem Bindemittel, bestehend aus

a) einem Epoxidharz mit durchschnittlich mehr als einer Epoxidgruppe pro Molekül und

b) Imidazolylverbindungen, welche herstellbar sind durch Umsetzung von

A) Imidazolylverbindungen der allgemeinen Formel (III)

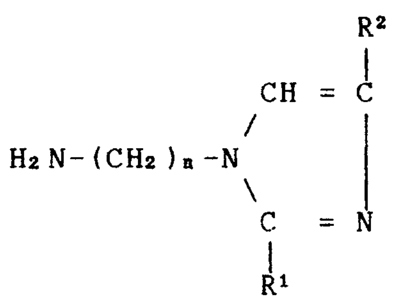

(III)

worin $R^1$ und $R^2$ unabhängig voneinander Wasserstoff, aliphatische oder aromatische Kohlenwasser stoff-Reste bedeuten und n = 2 oder 3 ist und

B) Diacrylaten der allgemeinen Formel (IV)

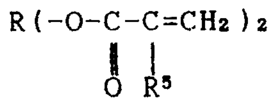

(IV)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist und $R^5$ Wasserstoff oder ein Methylrest sein kann im Molverhältnis von A : B = 1 : 2 bis 2 : 1 und gegebenenfalls weiterer Umsetzung dieser Additionsverbindungen mit

C)

1) Acrylsäure oder Acrylsäurederivaten der allgemeinen Formel (V)

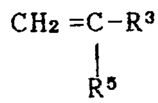

(V)

mit $R^3$ = -COOH, -CN, -CONH-$NH_2$, -$COOC_2H_4OH$,-$COOR^4$ mit $R^4$ = aliphatischer Kohlenwasserstoffrest mit 1 - 4 C-Atomen und $R^5$ Wasserstoff oder ein Methylrest sein kann, wenn das Additionsprodukt aus A) und B) freie Aminwasserstoffatome aufweist oder gegebenenfalls mit

2) Imidazolen der allgemeinen Formel (VI)

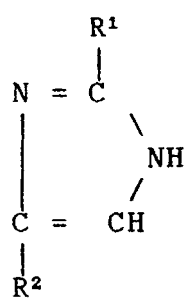

(VI)

in welcher $R^1$ und $R^2$ unabhängig voneinander die Bedeutung -H, $CH_3$-, $C_2H_5$, Phenyl haben können, wenn das Additionsprodukt aus A) und B) freie Doppelbindungen aufweist,

wobei die Verbindungen C) in solchen Mengen eingesetzt werden, daß alle reaktiven Aminwasserstoffatome oder Doppelbindungen der Additionsverbindungen aus A) und B) umgesetzt werden und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen, getränkt und gegebenenfalls bei erhöhter Temperatur in den halbfesten aber noch schmelzbaren Zustand (B-Zustand) überführt werden.

**5.** Verfahren zur Herstellung von Epoxidharzformkörpern, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formeln (I) und (II)

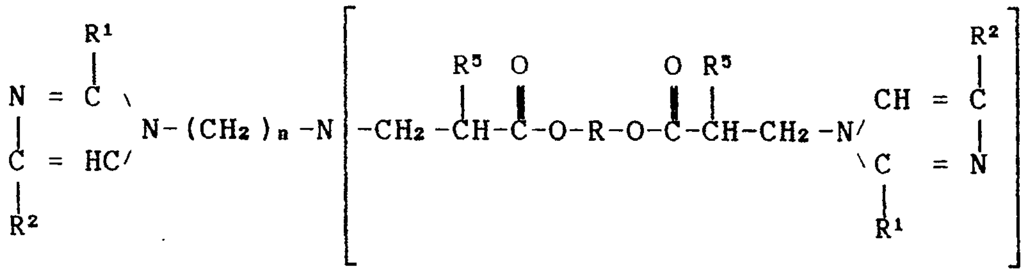

(I)

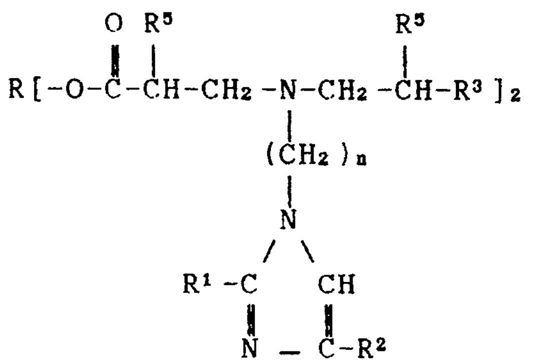

(II)

worin R ein zweiwertiger, gegebenenfalls verzweigter, gegebenenfalls Ethergruppen enthaltendcer aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann und gegebenenfalls

c) übliche Lösungsmitteln, Füllstoffen, Verstärkungs- und Einlagematerialien, Pigmenten, Hilfsstoffen und gegebenenfalls

d) üblichen stickstoffhaltigen heterocyclischen Aminverbindungen

getränkt und gegebenenfalls in den halbfesten aber noch schmelzbaren Zustand (B-Zustand) überführt werden und in zweiter Stufe die feuchten Laminate oder Prepregs unter Formgebung oder zwischen zu verklebende Substrate gebracht, unter Anwendung von Druck bei erhöhter Temperatur ausgehärtet werden.

**6.** Verfahren zur Herstellung von Epoxidharzformkörpern, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formel (I) und (II)

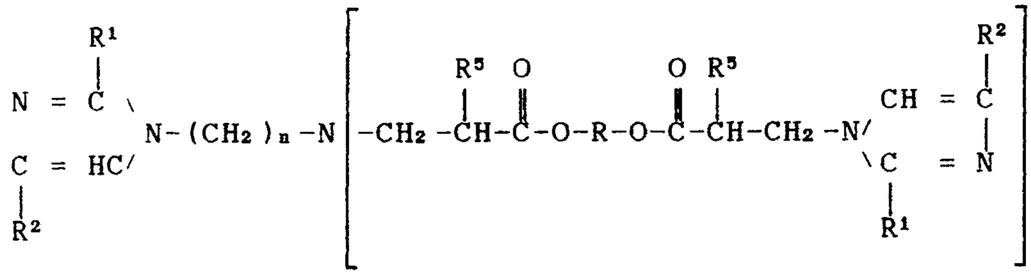

(I)

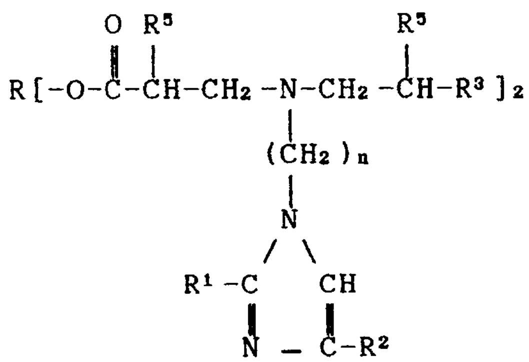

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste bedeuten, $R^3$ die Gruppen -COOH, -CN, $-COOC_2H_4-OH$, $-CONH-NH_2$, $-COOR^4$ sind mit $R^4$ = aliphatischer Kohlenwasserstoffrest mit 1 - 4 C-Atomen ist und n = 2 oder 3 ist und $R^5$ Wasserstoff oder ein Methylrest sein kann und gegebenenfalls

c) üblichen Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und in den festen und formstabilen Zustand überführt und in zweiter Stufe bei einer unterhalb der Erweichungstemperatur der gemäß Stufe 1 gebildeten Formkörper liegenden Temperatur ausgehärtet werden.

7. Verfahren zur Herstellung von faserverstärkten Trägermaterialien für den Elektrosektor durch Überführung von mit Bindemittel auf Basis von Epoxidharz und aminischen Härtungsmitteln imprägnierten Verstärkungsmaterialien in erster Stufe durch Anwendung von Temperatur und gegebenenfalls Druck in den B-Zustand und endgültiger Aushärtung bei erhöhter Temperatur, dadurch gekennzeichnet, daß als Härtungsmittel Verbindungen der allgemeinen Formeln (I) und (II)

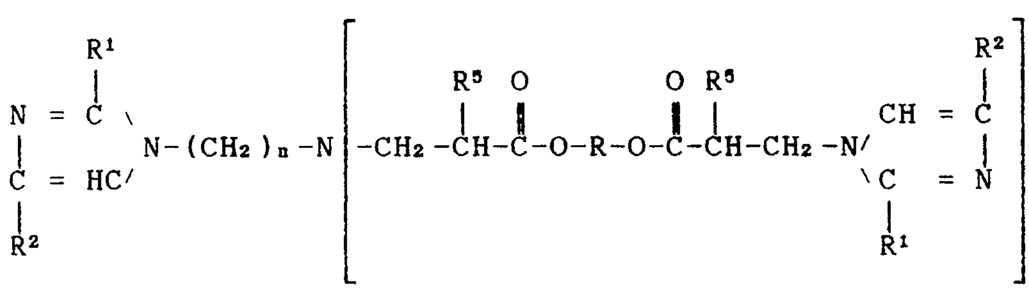

(I)

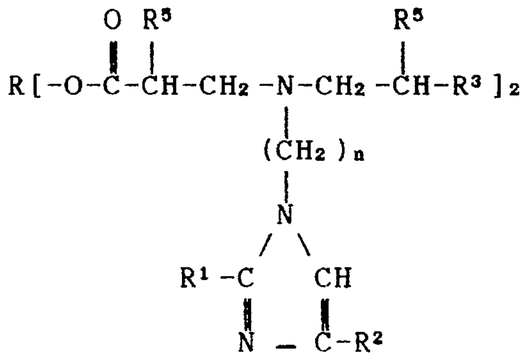

(II)

worin R ein zweiwertiger, gegebenenfalls verzweigter, gegebenenfalls Ethergruppen enthaltender aliphatischer, cyclischer oder alicyclischer Kohlenwasserstoffrest mit 2 - 20 C-Atomen ist, $R^1$ und $R^2$ unabhängig voneinander Wasserstoff, aliphatische oder aromatische Kohlenwasserstoffreste $C_2H_5$-bedeuten, $R^3$ die Gruppen -COOH, -CN, $-COOC_2H_4-OH$, $-CONH-NH_2$, $-COOR^4$ sind mit $R^4$ aliphatischer Kohlenwasserstoffrest mit 1 - 4 C-Atomen und n = 2 oder 3 ist und $R^5$ Wasserstoff oder ein Methylrest sein kann verwendet werden.

Europäisches Patentamt

# EUROPÄISCHER RECHERCHENBERICHT

Nummer der Anmeldung

**EP 91 10 6152**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 354 319 (SCHERING AG)<br>* Ansprüche *<br>– – – | 1-10 | C 07 D 233/61<br>C 08 G 59/50 |
| A | EP-A-0 239 246 (SHIKOKU CHEMICALS)<br>* Ansprüche *<br>– – – | 1-10 | |
| A | EP-A-0 024 119 (MOBIL OIL CORP.)<br>* Ansprüche *<br>– – – – – | 1-10 | |

RECHERCHIERTE SACHGEBIETE (Int. Cl.5)

C 07 D 233/00
C 07 D 249/00
C 08 G 59/00

Der vorliegende Recherchenbericht wurde für alle Patentansprüche erstellt

| Recherchenort | Abschlußdatum der Recherche | Prüfer |
|---|---|---|
| Den Haag | 25 Juni 91 | CHOULY J. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument